# EUROPÄISCHE PATENTSCHRIFT

(11) **EP 0 825 844 B1**
(45) Veröffentlichungstag und Bekanntmachung des Hinweises auf die Patenterteilung: **16.08.2000**
(21) Anmeldenummer: 96919549.4
(22) Anmeldetag: 10.05.1996
(51) Int. Cl.: A61F 13/00, A61M 27/00, A61M 1/00

(54) **VORRICHTUNG ZUR VAKUUMVERSIEGELUNG EINER WUNDE**
DEVICE FOR VACUUM-SEALING AN INJURY
DISPOSITIF PERMETTANT DE SUTURER UNE BLESSURE SOUS VIDE

(30) Priorität: 13.05.1995 DE 19517699
(43) Veröffentlichungstag der Anmeldung: 04.03.1998
(73) Patentinhaber: Fleischmann, Wim, 89182 Bernstadt (DE)
(72) Erfinder: Fleischmann, Wim, 89182 Bernstadt (DE)
(74) Vertreter: Mussgnug, Bernd, Dr.rer.nat.
(86) Internationale Anmeldenummer: DE9600869
(87) Internationale Veröffentlichungsnummer: WO9635401

(56) Entgegenhaltungen:
- EP-A- 0 182 248
- EP-A- 0 186 783
- WO-A-94/20041
- WO-A-96/05873
- DE-A- 3 523 843
- US-A- 5 279 550

## Beschreibung

Die Erfindung betrifft eine Vorrichtung zur Vakuumversiegelung einer Wunde gemäß dem Oberbegriff des Anspruchs 1.

Zur Versorgung großflächiger Wunden mit größeren Gewebsdefekten ist es aus WO 93/09727 bekannt, die Wunde mit einer Folie flächig zu überdecken und luftdicht abzuschließen. Ein unter die Folie eingelegter Drainageschlauch wird über einen Sekret-Auffangbehälter an ein Vakuumsystem angeschlossen. Dadurch wird im Wundbereich unter der Folie ein Unterdruck erzeugt, der die Gewebsproliferation begünstigt und das Wachstum von Bakterien verhindert. Bei größeren Wunden wird vorzugsweise eine poröse Schaumstoffeinlage in die Wunde eingelegt, in welche der Drainageschlauch eingezogen wird. Dadurch kann das Wundsekret gleichmäßiger über die gesamte Wundfläche abgesaugt werden.

Bei diesen bekannten Vorrichtungen wird bisher ein Unterdruck von etwa 80 kPa angewendet. Um diesen Unterdruck zu erzeugen, wird eine Vakuumpumpe an den Behälter zum Auffangen des Wundsekrets angeschlossen, so daß die Saugwirkung der Pumpe kontinuierlich über den Behälter einwirkt. Eine andere Möglichkeit besteht darin, einen evakuierten Behälter zu verwenden und diesen auszuwechseln, sobald der Unterdruck in dem Behälter unter einen bestimmten Wert abgebaut ist.

Der Erfindung zugrunde liegende Untersuchungen haben gezeigt, daß dieser bislang übliche Unterdruck insbesondere für chronische Wunden zu hoch ist. Druckwerte, die unter etwa 1/6 der bisher verwendeten Druckwerte, d. h. bei etwa 10 kPa liegen, scheinen sich auf den Heilungsprozeß günstiger auszuwirken. Der Einsatz kontinuierlich arbeitender Vakuumpumpen ist auf den stationären klinischen Aufenthalt des Patienten beschränkt. Der Einsatz von evakuierten Sekretauffangbehältern ist bei diesen geringen Unterdrücken mit erheblichen Schwierigkeiten verbunden. Bereits geringe Sekretmengen, die in dem Behälter aufgefangen werden, reichen aus, um einen erheblichen Druckabfall in dem Behälter zu verursachen. Ebenso führen praktisch kaum zu vermeidende minimale Undichtigkeiten in dem System zu einem Zusammenbruch des Unterdrucks.

Aus der US 3 779 243 ist eine Vorrichtung zur Drainage von Wunden bekannt, bei welcher ein Beutel zum Auffangen des Wundsekrets unter Unterdruck gesetzt wird. Hierzu sitzt der Beutel in einem Behälter aus einer Bodenplatte und einer Deckplatte, die durch eine verformbare Wandung verbunden sind. Zwischen die Bodenplatte und die Deckplatte sind Schraubendruckfedern eingesetzt, die die Bodenplatte und die Deckplatte auseinander drücken, um das Innenvolumen des Auffangbehälters zu vergrößern. Die Deckplatte und die Bodenplatte werden manuell gegen die Kraft der Druckfedern zusammengedrückt, um den Auffangbehälter zu evakuieren. Wird die Deckplatte freigegeben, so vergrößern die Druckfedern das Innenvolumen des Behälters, wodurch der Unterdruck in dem Auffangbehälter erzeugt wird.

Aus der US 3 809 086 ist eine Vorrichtung zur Drainage von Wunden bekannt, bei welcher zum Auffangen des Wundsekrets ein formstabiler Behälter verwendet wird, der an seiner einen Seite durch eine elastische Membran verschlossen ist. Die Membran wird manuell in den Behälter hineingedrückt, um diesen zu evakuieren.

Wird die Membran freigegeben, so erzeugt sie aufgrund ihrer elastischen Rückstellkraft einen Unterdruck in dem Behälter.

Bei diesen bekannten Vorrichtungen wird durch die Volumenvergrößerung des das Sekret aufnehmenden Behälters der Unterdruck zum Absaugen des Sekrets erzeugt. Für diese Pumpwirkung ist es notwendig, zunächst das Volumen des Behälters gegen die mechanische Rückstellkraft zu verkleinern, um den Behälter zu evakuieren. Aufgrund der Kennlinie der Rückstellfederkraft nimmt der in dem Behälter erzeugte Unterdruck mit zunehmender Füllung des Behälters ab. Es kann daher kein konstanter Unterdruck über die gesamte Behandlungsdauer aufrecht erhalten werden.

Weiter ist es aus der DE 35 23 843 C2 bekannt, eine Kolbenpumpe zu verwenden, um einen Unterdruck für die Wunddrainage zu erzeugen und aufrechtzuerhalten.

Der Erfindung liegt die Aufgabe zugrunde, eine Vorrichtung zur Vakuumversiegelung einer Wunde zur Verfügung zu stellen, bei welcher auch ein geringer Unterdruck in der Größenordnung von etwa 10 kPa oder weniger aufrechterhalten werden kann und die insbesondere auch unabhängig von einem stationären klinischen Aufenthalt ambulant eingesetzt werden kann.

Diese Aufgabe wird erfindungsgemäß gelöst durch eine Vorrichtung mit den Merkmalen des Anspruchs 1.

Vorteilhafte Ausführungen der Erfindung sind in den Unteransprüchen angegeben.

Erfindungsgemäß ist der Behälter zum Auffangen des Wundsekrets mit einer Pumpe vereinigt, mit welcher der Unterdruck in dem Behälter und damit in der versiegelten Wunde erzeugt wird. Die Vereinigung des Behälters mit der Pumpe macht den Patienten von dem stationären Vakuumsystem eines Krankenhauses unabhängig, so daß eine ambulante Versorgung von akuten und chronischen Wunden möglich ist. Durch die mit dem Behälter vereinigte Pumpe wird der Unterdruck in dem Behälter konstant auf dem gewünschten Wert gehalten. Der Patient selbst kann anhand einer an dem Behälter angebrachten Druckanzeige den jeweiligen Unterdruck überwachen.

Die Pumpe ist vollständig in dem Behälter integriert, so daß der Behälter und die Pumpe als Einmalartikel ausgebildet sind und nach Gebrauch gemeinsam entsorgt werden.

Der Behälter erfüllt selbst die Funktion der Pumpe. Der Behälter weist hierzu eine flexible Außenwandung auf, so daß er in axialer Richtung ausgedehnt werden kann, um entsprechend seiner Volumenvergrößerung einen Unterdruck zu erzeugen. Die axiale Volumenvergrößerung wird erzeugt, indem der Behälter pneumatisch ausgedehnt wird. Der das Behältervolumen ausdehnende pneumatische Druck bestimmt den in dem Behälter erzeugten Unterdruck. Auch hier ergibt sich entsprechend dem pneumatischen Druck, der das Behältervolumen vergrößert, ein vorgegebener Unterdruck, der über den gesamten Ausdehnungshub des Behälters konstant gehalten wird. Ein Korrigieren des Unterdrucks ist nur dann notwendig, wenn sich der Behälter über seinen gesamten Ausdehnungshub bewegt hat.

Die Folie zum Abdecken und Versiegeln der Wunde, die Schaumstoffeinlage, der Drainageschlauch und der Behälter mit der Pumpe werden vorzugsweise als Komplett-Set zur Verfügung gestellt, so daß der Arzt alle für die Wundversorgung erforderlichen Teile zur Verfügung hat.

Im folgenden wird die Erfindung anhand von in den Zeichnungen dargestellten Ausführungsbeispielen näher erläutert. Es zeigen
Fig. 1: schematisch eine erste Ausführung der Vorrichtung und
Fig. 2: den Behälter und die Pumpe in einer zweiten Ausführung.

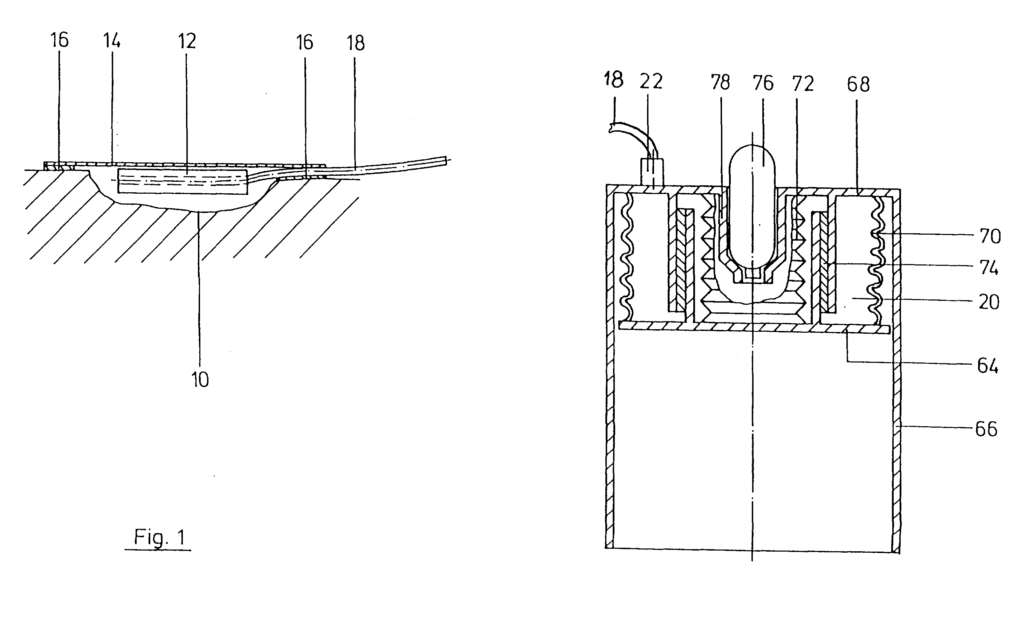

In Fig. 1 ist schematisch die gesamte Vorrichtung dargestellt. In die Wunde 10 des Patienten wird eine Einlage 12 aus einem offenporigen formstabilen Schaumstoff eingelegt. Die Wunde 10 mit der Einlage 12 wird durch eine luftdichte Kunststofffolie 14 überdeckt. Die Folie 14 greift über die Ränder der Wunde 10 und ist an ihrem Umfang durch geeignete Klebstoffe 16 abgedichtet auf der Haut des Patienten befestigt.

In die Einlage 12 ist das distale Ende eines Drainageschlauches 18 eingezogen. Der Drainageschlauch 18 weist an seinem in der Einlage 12 befindlichen Ende Öffnungen auf, so daß er über die Poren der Einlage 12 mit der Wunde kommuniziert.

Der Drainageschlauch 18 ist an seinem Außenumfang abgedichtet unter der Folie 14 herausgeführt.

An das proximale Ende des Drainageschlauches 18 ist ein Behälter 20 angeschlossen.

An dessen oberer Deckfläche ist ein Anschlußstutzen 22 mit Schlaucholive angeformt, auf den der Drainageschlauch 18 aufgeschoben wird. Gegebenenfalls kann der Drainageschlauch 18 auch einstückig an den Anschlußstutzen 22 angeformt sein. Der Drainageschlauch 18 steht über den Anschlußstutzen 22 mit dem Innenraum des Behälters 20 in Verbindung.

Zur Versorgung einer Wunde wird die Einlage 12 mit dem Drainageschlauch 18 in die Wunde 10 gelegt und durch die Folie 14 abgedeckt und luftdicht verschlossen. Der Behälter 20 wird evakuiert, vorzugsweise auf einen Unterdruck von ca. 10 kPa. Der in dem Behälter 20 herrschende Unterdruck pflanzt sich über den Drainageschlauch 18 und die poröse Schaumstoff-Einlage 12 fort und führt zu einer Evakuierung des Wundbereiches unter der Folie 14. Das von der Wunde 10 abgesonderte Sekret wird über die poröse Einlage 12 und den Drainageschlauch 18 in den Behälter 20 gesaugt und unten in dem Behälter gesammelt. Der Behälter 20 mit der integrierten Pumpe ist in der vorliegenden Ausführung als Einmal-Artikel ausgebildet. Wenn der Behälter 20 mit Wundsekret gefüllt ist oder wenn die Wundbehandlung abgeschlossen ist, wird der Behälter 20 mit der Pumpe und dem gesammelten Sekret entsorgt.

Der Boden 64 des Behälters 20 ist axial verschiebbar geführt in einer äußeren Zylinderwandung 66 des Behälters bzw. in einem entsprechenden aus achsparallelen Stäben gebildeten zylindrischen Käfig. Der Boden 64 ist an seinem äußeren Rand mit der oberen Deckfläche 68 des Behälters 20 durch einen flexiblen Folienschlauch 70 abgedichtet verbunden. Die obere Deckfläche 68, der Boden 64 und der Folienschlauch 70 umschließen somit einen Innenraum, dessen Volumen durch axiale Verschiebung des Bodens 64 veränderbar ist und an welchen der Drainageschlauch 18 über den Anschlußstutzen 22 angeschlossen werden kann.

Zwischen die obere Deckfläche 68 des Behälters 20 und den Boden 64 ist mittig ein zylindrischer geschlossener Balg 72 eingesetzt, der an seinem Außenumfang durch ein zwischen die Deckfläche 68 und den Boden 64 eingesetztes Teleskoprohr 74 geführt ist. Der Balg 72 kann pneumatisch unter Druck gesetzt werden, so daß er sich axial ausdehnt und den Boden 64 axial nach unten schiebt. Der pneumatische Druck kann beispielsweise durch eine Pumpe manuell oder elektrisch erzeugt werden. Eine leichte, platzsparende und komfortable pneumatische Betätigung des Balges 72 wird vorzugsweise erreicht durch eine Gaspatrone 76, die in eine entsprechende Aufnahme 78 der oberen Deckfläche 68 einsetzbar ist und über ein Ventil mit dem Inneren des Balges 72 in Verbindung kommt.

In dieser Ausführung arbeitet die Vorrichtung in folgender Weise. Der Boden 64 wird vollständig eingeschoben, so daß das Innenvolumen des Behälters 20 verkleinert wird. Dann wird der Drainageschlauch 18 an den Anschlußstutzen 22 angeschlossen. Nun wird die Gaspatrone 76 in die Aufnahme 78 eingesetzt, so daß Gas unter Druck in den Balg 72 einströmt. Der Balg 72 dehnt sich aus und verschiebt den Boden 64 nach unten, wodurch sich das Innenvolumen des Behälters 20 vergrößert. Die Vergrößerung des Innenvolumens erzeugt einen Unterdruck in dem Behälter 20. Es bildet sich ein Gleichgewichtszustand aus, bei welchem der Unterdruck in dem Behälter 20 den Gasdruck in dem Balg 72 kompensiert. Verringert sich der Unterdruck in dem Behälter 20 durch Undichtigkeit des Systems und durch Sekreteinstrom, so wird der Boden 64 durch den Gasdruck in dem Balg 72 weiter verschoben, so daß der Gleichgewichtsunterdruck konstant aufrechterhalten bleibt.

Hat sich der Boden 64 bis in seine untere Endstellung verschoben, wird die Gaspatrone 76 herausgenommen und der Behälter 20 kann über den Anschlußstutzen 22 durch Eindrücken des Bodens 64 entleert oder entsorgt und durch einen neuen Behälter 20 ersetzt werden. Nach dem Entleeren des Behälters bzw. dem Austausch gegen einen neuen Behälter 20 wird die Gaspatrone 76 wieder eingesetzt und der Absaugvorgang kann fortgesetzt werden.

Der sich in der Zylinderwandung 66 verschiebende Boden 64 dient gleichzeitig als Anzeige dafür, wann der Behälter 20 entleert bzw. ausgetauscht werden muß.

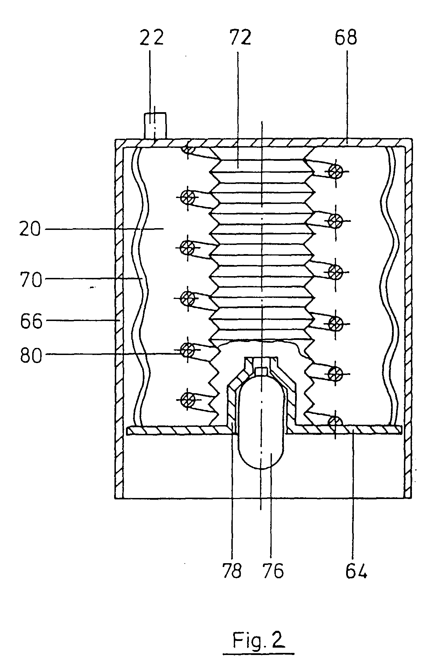

Fig. 2 zeigt eine zweite Ausführung, die eine Abwandlung der Ausführung der Fig. 1 darstellt.

Im Gegensatz zum Ausführungsbeispiel der Fig. 1 ist in dieser zweiten Ausführung die Aufnahme 78 für die Gaspatrone 76 im Boden 64 angeordnet und anstelle eines Teleskoprohres ist eine Schraubenfeder 80 zwischen den Boden 64 und die obere Deckfläche 68 eingesetzt, um den Balg 72 gegen eine seitliche Auslenkung zu führen. In Fig. 1 ist der Behälter in seiner Ausgangsstellung zu Beginn des Absaugvorgangs dargestellt, in welcher der Boden 64 vollständig eingeschoben ist, während in Fig. 6 die Endstellung gezeigt ist, in welcher sich der Boden 64 in seiner unteren Endstellung befindet und der Saugvorgang beendet ist. Im übrigen entspricht die Ausführung der Fig. 2 der Ausführung der Fig. 1.

## Patentansprüche

1. Vorrichtung zur Vakuumversiegelung einer Wunde (10), mit einer Folie (14) zum flächigen Überdecken und luftdichten Abschließen der Wunde (10), mit einem Drainageschlauch (18), dessen distales Ende unter die Folie (14) in die Wunde (10) einlegbar ist, und mit einem Behälter (20) zum Auffangen des Wundsekrets, der unter Unterdruck setzbar und an das proximale Ende des Drainageschlauches (18) anschließbar ist, wobei der Behälter (20) durch einen Boden (64), eine Deckfläche (68) und eine verformbare Wandung (70) gebildet ist und wobei zwischen den Boden (64) und die Deckfläche (68) ein Balg (72) eingesetzt ist, der unter pneumatischen Druck setzbar ist und dadurch das Innenvolumen des Behälters (20) zur Erzeugung des Unterdruckes vergrößert.

2. Vorrichtung nach Anspruch 1, dadurch gekennzeichnet, daß in die Deckfläche (68) oder den Boden (64) eine Gaspatrone (76) einsetzbar ist, die mit dem Inneren des Balges (72) in Verbindung kommt.

3. Vorrichtung nach Anspruch 1 oder 2, dadurch gekennzeichnet, daß die verformbare Wandung ein flexibler Folienschlauch (70) ist und der Boden (64) in einer Zylinderwandung (66) oder einem Käfig des Behälters (20) geführt ist.

4. Vorrichtung nach Anspruch 3, dadurch gekennzeichnet, daß die Stellung des Bodens (64) als Druckanzeige dient.

## Claims

1. A device for vacuum sealing a wound (10), with a film (14) for the flat covering and air-tight sealing of the wound (10), with a drainage tube (18), the distal end of which can be inserted beneath the film (14) into the wound (10), and with a basin (20) for collecting the wound exudation which can be placed under partial vacuum and can be connected to the proximal end of the drainage tube, wherein the basin (20) is formed by a base (64), an upper surface (68) and a deformable wall (70) and wherein inserted between the base (64) and the upper surface (68) are bellows (72), which can be placed under pneumatic pressure and as a results increase the internal volume of the basin (20) for the production of the partial vacuum.

2. A device according to Claim 1,
**characterised in that** a gas cartridge (76) that comes into communication with the interior of the bellows (72) can be inserted into the upper surface (68) or the base (64).

3. A device according to Claim 1 or 2,
**characterised in that** the deformable wall is a flexible film tube (70) and the base (64) is guided in a cylinder wall (66) or a cage of the basin (20).

4. A device according to Claim 3,
**characterised in that** the position of the base (64) serves as a pressure indicator.

## Revendications

1. Dispositif de suture sous vide d'une blessure (10) avec une feuille (14) pour le revêtement de surface et la fermeture étanche à l'air de la blessure (10), avec un tuyau de drainage (18) dont l'extrémité distale peut être insérée sous la feuille (14) dans la blessure (10), et avec un récipient (20) qui peut être mis sous dépression pour collecter les sécrétions de la blessure et peut être raccordé à l'extrémité proximale du tuyau de drainage (18), dans lequel :
- le récipient (20) est formé par un fond (64), par une surface de recouvrement (68) et une paroi déformable (70), et
- entre le fond (64) et la surface de recouvrement (68) est inséré un soufflet (72) qui est réglable à une pression pneumatique et grâce auquel on peut accroître le volume interne du récipient (20) pour produire la dépression.

2. Dispositif selon la revendication 1,
caractérisé en ce qu'
on peut insérer dans la surface de recouvrement (68) ou le fond (64) une cartouche de gaz (76) qui vient en liaison avec l'intérieur du soufflet (72).

3. Dispositif selon la revendication 1 ou 2,
caractérisé en ce que
la paroi déformable est un tube en feuille souple (70) et en ce que le fond (64) est conduit dans une paroi cylindrique (66) ou une cage du récipient (20).

4. Dispositif selon la revendication 3,
caractérisé en ce que
la position du fond (64) sert d'indicateur de pression.
